# EUROPÄISCHE PATENTSCHRIFT

(11) **EP 4 228 532 B1**
(45) Veröffentlichungstag und Bekanntmachung des Hinweises auf die Patenterteilung: **20.08.2025**
(21) Anmeldenummer: 21801435.5
(22) Anmeldetag: 18.10.2021
(51) Int. Cl.: A61B 17/28, A61B 90/00, A61B 17/00

(54) **MEDIZINISCHES INSTRUMENT MIT GLEICHMÄSSIGER GANGHÄRTE**
MEDICAL INSTRUMENT WITH CONSISTENT SMOOTHNESS OF ACTION
INSTRUMENT MÉDICAL À RÉGULARITÉ D'ACTION CONSTANTE

(30) Priorität: 19.10.2020 DE 102020127497
(43) Veröffentlichungstag der Anmeldung: 23.08.2023
(73) Patentinhaber: Aesculap AG, 78532 Tuttlingen (DE)
(72) Erfinder: BARTHELMES, Sven, 78576 Emmingen-Liptingen (DE); LOESDAU, Holger, 78573 Wurmlingen (DE); STARK, Christian, 78532 Tuttlingen (DE); KNECHT, Markus, Penang, 11700 (MY)
(74) Vertreter: Winter, Brandl - Partnerschaft mbB
(86) Internationale Anmeldenummer: PCT/EP2021/078822
(87) Internationale Veröffentlichungsnummer: WO 2022/084256

(56) Entgegenhaltungen:
- EP-A1- 2 594 210
- WO-A1-2018/166989
- DE-A1- 10 101 425
- DE-A1- 102008 058 207
- DE-A1- 102016 111 892
- DE-A1- 102016 116 624

## Beschreibung

### Technisches Gebiet

Die vorliegende Erfindung betrifft ein medizinisches / medizintechnisches, insbesondere chirurgisches, Instrument mit einer ersten Instrumentenbranche, die einen ersten Lagerabschnitt mit zumindest einer ersten Lauffläche / Gleitfläche aufweist, und mit einer zweiten Instrumentenbranche, die einen zweiten Lagerabschnitt mit zumindest einer zweiten Lauffläche / Gleitfläche aufweist, an / auf dem die erste Lauffläche flächig schwenkgleitend aufliegt / anliegt / aneinander liegt, so dass die erste Instrumentenbranche zu der zweiten Instrumentenbrachen relativbewegbar und um eine Schwenkachse schwenkbar ist.

### Stand der Technik

Medizinische Instrumente, wie etwa chirurgische Klemmen mit zwei zueinander schwenkbaren Instrumentenbranchen, weisen als Schwenkgelenk üblicherweise eine Lagerung in Form eines Durchsteckschlusses auf, bei welchem ein männliches Durchsteckteil in einem weiblichen Durchsteckkasten aufgenommen ist und an jeweils zwei direkt anliegenden Laufflächen der beiden Branchen schwenkgleitend in eine geschlossene, klemmende Stellung und in eine geöffnete Stellung schwenkbar ist. Diese unmittelbar aufeinanderliegenden Laufflächen sind meist über den gesamten Lagerabschnitt plan oder unter einem geringen Winkel nach außen abfallend ausgebildet. Der Durchsteckschluss weist kleine Spaltmaße sowie scharfe oder ausgeprägt kantige Innenecken oder Kanten auf, die bei einem Öffnen und Schließen des medizinischen Instruments über die jeweils gegenüberliegende, direkt anliegende Lauffläche als auch die Kanten dieser Lauffläche schleifen. Zum einen ändert sich dabei eine Länge der aufsetzenden Kante über eine Öffnungs- bzw. Schließbewegung hinweg und zum anderen sind die Spaltmaße zwischen den Laufflächen ungleich oder ansteigend. In Folge tritt ein ungleicher Gang mit einhergehender unterschiedlicher Ganghärte im Bewegungsbereich der Relativbewegung auf. Insbesondere ändert sich also je nach Relativstellung / Öffnungsstellung der Instrumentenbranchen zueinander eine Reibung bzw. Reibkraft zwischen den Lagerabschnitten der Instrumentenbranchen, was in einem ungleichen Gang resultiert. Ein solcher Effekt eines ungleichen Gangs lässt sich beispielsweise bei einer chirurgischen Schere beobachten, welche mit zunehmender Schließstellung eine höhere Schließkraft erfordert.

Die US 3,459,187 A etwa offenbart ein medizinisches Instrument in Form einer chirurgischen Klemme mit zwei zueinander schwenkbaren Instrumentenbranchen in Durchsteckkonfiguration. In einem Schwenkgelenksbereich steht ein männliches Durchsteckteil durch einen weiblichen Durchsteckkasten hindurch und liegt in diesem gesamtflächig an den Innenwänden an. Bei einer Öffnungs- und Schließbewegung gleiten, unter Reibung, der männliche und der weibliche Lagerabschnitt aufeinander ab. Dementsprechend ändert sich eine Ganghärte bei einem schwenkbaren Positionieren der Instrumentenbranchen zueinander.

Die WO 2018 / 166 989 A1 offenbart eine additiv gefertigte chirurgische Klemme mit zwei zueinander schwenkbaren Instrumentenbranchen. Eine Instrumentenbranche weist dabei einen in Richtung der anderen Instrumentenbranche erhebenden Führungsvorsprung auf, der zum schwenkenden Positionieren der Instrumentenbranche in eine entsprechend ausgebildete kreisbogenförmige Vertiefung der anderen Instrumentenbranche eingreift. Auch bei dieser chirurgischen Klemme unterscheidet sich eine Ganghärte im Bewegungsbereich, insbesondere bei einem Öffnen der Klemme aus der geschlossenen Stellung heraus. Auch ist eine Bauteilstabilität im geöffneten Zustand vergleichsweise gering und bei falscher Handhabe bruchanfällig.

Die DE 101 01 425 A1 offenbart ein medizinisches Instrument mit zwei Instrumentenbranchen, die um eine Drehachse gegeneinander schwenkbar sind, wobei im Bereich des Lagers eine Art wellenförmiger Vorsprung vorgesehen ist.

Die EP 2 594 210 A1 offenbart ebenfalls ein medizinisches Instrument mit zwei schwenkbaren Instrumentenbranchen, wobei zwischen diesen eine Tellerfeder eingesetzt ist.

Die DE 10 2008 058 207 A1 zeigt eine Handgriffvorrichtung für ein Chirurgie-Werkzeug, wobei über eine Kopplungseinheit zwei Instrumentenbranchen drehbar miteinander gelagert sind. Hierbei ist ein Zentrierelement in Form eines Stifts vorgesehen.

Die DE 10 2016 111 892 A1 offenbart ein Instrument mit wellenförmigen Laufflächen, bei denen Kontaktlinien und Kontaktpunkte vorliegen.

Die DE 10 2016 116624 A1 offenbart ein medizinisches Instrument mit einer Lagerung nach Art eines Bajonettverschlusses, wobei ein Lagerelement der Lagerung mindestens einen vorzugsweise mehrere Vorsprünge bzw. Überstände aufweist.

### Zusammenfassung der Erfindung

Es ist daher die Aufgabe der Erfindung, die Nachteile aus dem Stand der Technik zu vermeiden oder wenigstens zu mindern und insbesondere ein medizinisches Instrument zur Verfügung zu stellen, das eine Ganghärte im gesamten Bewegungsbereich bzw. in jeder Relativstellung der Instrumentenbranchen zueinander besonders gleichmäßig hält. Ferner soll eine Herstellung und Produktion, insbesondere ein Montageprozess, vereinfacht werden. Ebenfalls ist eine weitere Aufgabe der Erfindung, dass produktionsbedingte Oberflächenfehler wie etwa Kratzer auf einer sichtbaren Lauffläche minimiert werden sollen. Zudem soll eine Reinigbarkeit bzw. Sterilisierbarkeit verbessert werden.

Die Aufgaben und Ziele der Erfindung bei einem gattungsgemäßen medizinischen Instrument werden erfindungsgemäß durch die Merkmale des Anspruchs 1 gelöst.

Grundsätzlich sieht die Erfindung also vor, dass die (zumindest eine) erste Lauffläche und/oder die (zumindest eine) zweite Lauffläche im Bereich der Schwenkachse bzw. um die Schwenkachse herum gegenüber ihrem (restlichen) Lagerabschnitt in Richtung der Schwenkachse des medizinischen Instruments nach außen und damit zur zweiten bzw. ersten Lauffläche hin abgesetzt / versetzt ist. Durch diese Konfiguration wird ein Vorsprungs-Abschnitt ausgebildet, der speziell die, insbesondere auf einer Seite gesamte, Lauffläche definiert und über die Erhebung durch den Absatzabschnitt oder das Absatzelement einen (kleinen) Spalt zwischen den (restlichen) einander zugewandten Wandflächen des Lagerabschnitts ausbildet. Da in Folge nur die abgesetzte Lauffläche um die Schwenkachse herum direkt in Kontakt mit der entsprechend gegenüberliegenden Lauffläche steht, kann diese speziell gestaltet und etwa besonders gut bearbeitet und für den schwenkgleitenden Einsatz vorbereitet werden. Es kann an der Lauffläche durch lokale Bearbeitung eine besonders hohe Oberflächenqualität dieser Lauffläche hergestellt werden. Aufgrund des erzeugten Spalts im restlichen Teil / Abschnitt der Lagerabschnitte sind die produktionsbedingten unterschiedliche Spaltmaße nicht mehr relevant und ohne Einfluss, so dass ein Gang bzw. eine Ganghärte im gesamten Bewegungsfeld weiter harmonisiert bzw. über die gesamte Relativbewegung hinweg einem gleichmäßigen, konstanten Gang angenähert wird. Die Konfiguration mit dem Versatzabschnitt oder dem Versatzelement stellt also einen noch gleichmäßigeren Gang sicher, erhöht eine Bauteilstabilität und optimiert ein Kapillarverhalten während einer elektrochemischen Bearbeitung durch, insbesondere über den Lagerabschnitt entlang gleichbleibende, Spaltmaße. Durch das besondere Design des Instruments, bei welchem die Lauffläche gegenüber dem zugehörigen Lagerabschnitt nach außen abgesetzt ist bzw. gegenüber dem Lagerabschnitt zu der anderen Instrumentenbranche hin (nach außen) vorsteht und die schwenkgleitende Kontaktfläche zu der anderen Instrumentenbranche hin bildet, kann diese Lauffläche gezielt bearbeitet werden, um lokal eine besonders hohe Oberflächengüte (insbesondere relativ gesehen gegenüber der verbleibenden Oberfläche des Lagerabschnitts) zu erreichen. Durch die begrenzte und geometrisch genau definierte schwenkgleitende Lauffläche wird eine einfache und effiziente Herstellung des medizinischen Instruments ermöglicht und erreicht.

Insbesondere kann der Absatzabschnitt oder das Absatzelement wärmebehandelt sein, um eine andere Eigenschaft des Materials des Absatzabschnitts oder Absatzelements, insbesondere der Lauffläche, gegenüber dem verbleibenden Part des medizinischen Instruments zu erreichen.

Auch kann etwa insbesondere der Absatzabschnitt oder das Absatzelement, insbesondere die Lauffläche, oberflächenbehandelt sein, vorzugsweise oberflächengehärtet und/oder nitriert und/oder phosphatiert sein, um etwa eine Reibung und einen Verschleiß lokal zu mindern. Vorzugsweise ist nur der Absatzabschnitt oder das Absatzelement, insbesondere die Lauffläche, oberflächenbehandelt (und der restliche Teil des Instruments nicht), um eine kostengünstige Produktion mit lokaler hoher Oberflächengüte zu erreichen.

Zudem kann insbesondere der Absatzabschnitt oder das Absatzelement, insbesondere die Lauffläche, gehärtet sein und eine höhere Härte als der restliche Lagerabschnitt, insbesondere als das restliche medizinische Instrument, aufweisen.

Vorzugsweise kann ferner auch der Absatzabschnitt oder das Absatzelement eine die Lauffläche bildende biokompatible Beschichtung aufweisen, insbesondere mit Polymeren, insbesondere Fluorpolymere, und/oder mit PEEK und/oder mit Titan.

Insbesondere im Falle, dass sowohl der erste Lagerabschnitt als auch der zweite Lagerabschnitt einen Absatzabschnitt oder Absatzelement aufweisen, deren Stirnseiten jeweils die Laufflächen bilden, müssen nur diese einer besonderen Bearbeitung unterzogen werden, um insbesondere eine niedrige Rauigkeit für eine gleichmäßige Schwenkbewegung sicherzustellen. Wenn beide Stirnseiten zudem noch plan sind, so kann eine gleichmäßige Fläche mit nur sehr geringen Unebenheiten erzielt werden. Eine Produktion mit entsprechender spezieller Bearbeitung der Laufflächen kann noch einfacher und kostengünstiger erfolgen und eine Ergonomie des Instruments weiter verbessert werden.

Eine solche Ausgestaltung bringt die Vorteile einer höheren mechanischen Belastbarkeit, einer prozesssicheren maschinellen Herstellung, einer Erhöhung eines Mechanisierungsgrads oder Automatisierungsgrads in der Herstellung sowie einhergehend bessere Reinigungseigenschaften. Durch die geringfügige Erhöhung der Spaltmaße wird auch Raum für weitere strukturelle Anpassungen wie etwa zusätzliche Kantenverrundung geschaffen. Auch wird durch den, insbesondere gleichleibenden, Spalt bzw. das Spaltmaß zwischen den einander gegenüberliegenden "Innenflächen" der Lagerabschnitte ein optimiertes Reinigungsergebnis erzielt. Ebenso wird auch eine elektrochemische Bearbeitung optimiert.

Gemäß der Erfindung hat also der erste Lagerabschnitt und/oder der zweite Lagerabschnitt im Bereich der Schwenkachse einen Absatzabschnitt oder ein Absatzelement mit einer die zugehörige Lauffläche aufweisenden oder bildenden Stirnseite, der/das in Richtung einer Schwenkachse des medizinischen Instruments gegenüber dem zugehörigen Lagerabschnitt nach außen abgesetzt / versetzt ist bzw. vorsteht, so dass auch die zugehörige, insbesondere gesamte, Lauffläche gegenüber dem zugehörigen Lagerabschnitt nach außen abgesetzt ist. Insbesondere hat zumindest der erste Lagerabschnitt im Bereich der Schwenkachse zumindest einen Absatzabschnitt oder ein Absatzelement mit einer die erste Lauffläche aufweisenden, insbesondere bildende, Stirnseite, der/das in Richtung der Schwenkachse gegenüber dem ersten Lagerabschnitt nach außen abgesetzt ist, so dass die erste Lauffläche gegenüber dem ersten Lagerabschnitt nach außen zu der zweiten Lauffläche hin abgesetzt ist.

Der Begriff schwenkgleitend bedeutet hierbei, dass die beiden Laufflächen aufeinander flächig abgleiten und dabei relativ zueinander eine Schwenkbewegung um eine Schwenkachse vollziehen. Dies tritt beispielsweise dann auf, wenn eine erste plane (Wand-)Fläche, etwa horizontal, auf einer zweiten planen (Wand-)Fläche unmittelbar aufliegt und diese beiden Flächen gegeneinander gedreht werden, so dass diese schwenkgleitend aufeinander aufliegen.

Die Stirnseite ist hierbei eine Seite des Absatzabschnitts oder Absatzelements, die zu dem anderen Lagerabschnitt bzw. der anderen Lauffläche hin vorsteht, um an der anderen Lauffläche flächig aufzuliegen. Zumindest ein Teilbereich der Stirnseite bildet dabei die Lauffläche aus. Insbesondere steht die Schwenkachse senkrecht auf der Stirnseite. Die Stirnseite ist also dem Lagerabschnitt abgewandt bzw. von diesem (nach außen) wegweisend.

Der Ausdruck "im Bereich der Schwenkachse" definiert, dass im Bereich um die Schwenkachse herum, und damit insbesondere in einem mittigen, zentralen Abschnitt des Lagerabschnitts, der Absatzabschnitt bzw. das Absatzelement angeordnet ist. Die lateralen Abschnitte des Lagerabschnitts (also außerhalb des Bereichs der Schwenkachse) hingegen weisen keinen Absatzabschnitt oder Absatzelement auf. Die ersten und zweiten Lagerabschnitte sind dort beabstandet voneinander und dienen folglich auch nicht als Lauffläche.

Der Begriff "abgesetzt" definiert, dass zwischen der Lauffläche und dem restlichen Lagerabschnitt ein Versatz in Richtung der Schwenkachse vorliegt. Ähnlich eines Plateaus, einer Plattform oder einer Erhebung, bei der die obere Plateau-/Pattform-/Erhebungs-Fläche eine gegenüber dem Boden abgesetzte Fläche bildet, ist vorliegend die Lauffläche gegenüber einer Basisfläche des Lagerabschnitts abgesetzt.

Gemäß einem weiteren, gegebenenfalls zusammen mit dem Oberbegriff unabhängig beanspruchbaren, Aspekt der Erfindung sind die erste Lauffläche und die zweite Lauffläche derart ausgebildet und aufeinander abgestimmt, dass bei gleitender Relativbewegung der ersten Instrumentenbranche zu der zweiten Instrumentenbranche eine Flächengröße einer Kontaktfläche der zwei aufeinanderliegenden Laufflächen in jeder Relativstellung / Relativposition gleich (groß) ist, um eine Ganghärte im gesamten Bewegungsbereich der Relativbewegung gleichmäßig zu halten. Im Gegensatz zum Stand der Technik ändert sich durch die technische Konfiguration des medizinischen Instruments und der aufeinanderliegenden Laufflächen also eine Flächengröße der Kontaktfläche der beiden Laufflächen, also die Fläche der beiden gegenüberliegenden Laufflächen, die in der jeweiligen Relativstellung direkt aufeinander aufliegen, nicht, was in einer gleichbleibenden Reibungskraft und einhergehend in einer gleichbleibenden Ganghärte resultiert. Durch eine solche Konfiguration mit entsprechender Abstimmung der Laufflächen aufeinander ist die Kontaktfläche zwischen den Laufflächen in jeder Stellung gleich groß. Dies stellt einen gleichmäßigen Gang entlang des gesamten Bewegungsfeldes sicher und erhöht eine Bauteilstabilität. Durch die gleichbleibende Kontaktfläche (Auflagefläche) herrschen in jeder (Öffnungs-) Stellung die gleichen Reibungskräfte. Der Begriff Relativstellung im gesamten Bewegungsfeld bezeichnet dabei jede mögliche relative Positionierung bzw. Stellung der beiden Instrumentenbranchen zwischen einer Schließstellung und einer maximalen Öffnungsstellung zueinander.

Die erste Lauffläche und/oder die zweite Lauffläche ist drehsymmetrisch / rotationssymmetrisch, insbesondere kreisförmig oder ringförmig mit kreisförmigem Außendurchmesser, ausgebildet, welche insbesondere in jeder Relativstellung des gesamten Bewegungsbereichs in ihrer gesamten Fläche auf der jeweils anderen Lauffläche aufliegt und die Kontaktfläche bildet. Insbesondere durch die kreisförmige Ausbildung der Lauffläche, die um die Schwenkachse konzentrisch angeordnet ist, liegt bei einer Schwenkbewegung die kreisförmige Umfangskante tangential zu einer Bewegungsrichtung, so dass ein Einfluss, insbesondere ein Reibeinfluss, durch die Umfangskante minimiert wird. So herrschen in jeder Relativstellung die gleichen Reibkräfte und insbesondere berührt keine Bauteilkante die Lauffläche. Die gegenüberliegende, anliegende Lauffläche kann dann etwa flächig, insbesondere plan flächig, über den gesamten Lagerabschnitt gestaltet sein. Aufgrund der kreisförmigen Gestaltung der einen Lauffläche (auf zumindest einer Seite) wird eine Reibungskraft und damit eine Ganghärte gleichgehalten.

Vorteilhafte Ausführungsformen sind in den Unteransprüchen beansprucht und werden insbesondere nachfolgend erläutert.

Insbesondere weist zumindest der erste Lagerabschnitt zumindest eine abgesetzte erste Lauffläche auf, welche unabhängig der Öffnungsstellung bzw. Relativstellung eine gleichbleibend große Kontaktfläche mit der zweiten Lauffläche hat. Hierdurch resultiert eine besonders gleichmäßige Ganghärte über das gesamte Bewegungsfeld des medizinischen Instruments hinweg, bei dem auch noch gute Reinigungseigenschaften vorliegen und eine einfache Herstellung erfolgen kann.

Gemäß einer bevorzugten Ausführungsform kann in jeder Relativstellung die gesamte erste(n) Lauffläche(n) auf der/den zweiten Lauffläche(n) vollständig aufliegen. Dadurch dass die (zumindest eine) erste Lauffläche stets vollumfänglich auf der (zumindest einen) zweiten Lauffläche vollumfänglich aufliegt, ändert sich auch eine Größe einer Kontaktfläche nicht und eine Ganghärte bleibt gleich. Dadurch kommt die erste Lauffläche auch nicht in ein Sichtfeld eines Anwenders. Mit anderen Worten weist zumindest der erste Lagerabschnitt eine solche erste Lauffläche auf, welche unabhängig der Öffnungsstellung der beiden Branchen zueinander zu der zweiten Lauffläche eine gleichbleibend große Kontaktfläche hat. Insbesondere liegen in jeder Relativstellung die gesamte erste Lauffläche auf der gesamten zweiten Lauffläche auf. So beschränken sich insbesondere die ersten und/oder zweiten Laufflächen nur auf den nicht sichtbaren Teil der "Schlussfläche" bzw. Kontaktfläche und geraten in keiner (Relativ-)Stellung der Instrumentenbranchen zueinander in ein Sichtfeld.

Vorzugsweise kann die erste Lauffläche und/oder die zweite Lauffläche plan, also in einer Ebene liegend, ausgebildet sein. Dadurch ist eine besonders einfache Herstellung und gute schwenkgleitende Eigenschaft gegeben.

Insbesondere weist die zumindest eine erste und/oder zumindest eine zweite Lauffläche eine zusammenhängende Fläche auf, so dass auch eine Kontaktfläche eine zusammenhängende Fläche bildet. So werden zusätzliche Reibungseinflüsse minimiert und insbesondere eine Größe der jeweiligen Lauffläche maximiert, um eine Bauteilstabilität zu erhöhen.

Gemäß einem Aspekt der Erfindung kann der erste Lagerabschnitt und/oder der zweite Lagerabschnitt einen, insbesondere zylinderförmigen oder hohlzylinderförmigen, Sockel mit einer planen, die Lauffläche bildenden Stirnseite als Absatzabschnitt oder Absatzelement aufweisen. Insbesondere beträgt ein Verhältnis von einem Außendurchmesser des zylinderförmigen oder hohlzylinderförmigen Sockels zu einer Höhe des Sockels (in Richtung der Schwenkachse ausgehend vom Fuß des Sockels) und damit zu der zugehörigen Lauffläche zwischen 10:1 bis 200:1, vorzugsweise zwischen 50:1 und 100:1. Ähnlich eines flachen, planen Plateaus bildet die plane Stirnseite die Lauffläche aus. Vorzugsweise kann konzentrisch in dem Sockel ein Durchgangsloch ausgebildet sein.

Gemäß einer Ausführungsform kann der Absatzabschnitt stoffeinstückig an / mit dem zugehörigen Lagerabschnitt, insbesondere mit der zugehörigen Instrumentenbranche ausgebildet sein. Bei einer solchen integralen Herstellung werden zusätzliche Ritzen und Spalte vermieden, wodurch eine Reinigungseigenschaft weiter verbessert und eine Bauteilstabilität erhöht werden. Auch wird ein Herstellungsprozess vereinfacht, da eine separate Fertigung und Montage entfällt.

Gemäß einer weiteren bevorzugten Ausführungsform kann das medizinische Instrument eine Durchsteckkonfiguration / einen Durchsteckschluss aufweisen, bei der der erste Lagerabschnitt ein männlicher Lagerabschnitt in Form eines Durchsteckteils und der zweite Lagerabschnitt ein eine Durchgangsöffnung aufweisender weiblicher Lagerabschnitt in Form eines Durchsteckkastens ist, bei welcher der männliche Lagerabschnitt die Durchgangsöffnung des weiblichen Lagerabschnitts durchgreift und an zwei abgewandten Laufflächen / Auflagefläche / Anlageflächen des männlichen Lagerabschnitts an zwei zugewandten Laufflächen des weiblichen Lagerabschnitts schwenkgleitend anliegt. Eine Durchsteckkonfiguration ist besonders stabil und gut in der Herstellung als auch in der Reinigung bzw. Sterilisierung.

Vorzugsweise kann der männliche Lagerabschnitt zwei abgewandte, kreis- oder ringförmige, koaxial zueinander angeordnete Sockel mit planer Stirnseite aufweisen, welche die beiden einander abgewandten ersten (männlichen) Laufflächen des männlichen Lagerabschnitts bilden. Eine solche Ausgestaltung auf beiden Seiten des männlichen Abschnitts trägt beidseits zur gleichmäßigen Ganghärte bei und erhöht gleichzeitig eine Bauteilstabilität und eine Reinigungseigenschaft.

Gemäß der Erfindung sind die Laufflächen derart ausgebildet und aufeinander abgestimmt, dass auf der einen Lauffläche stets nur eine Fläche der anderen Lauffläche kantenlos aufliegt. Mit anderen Worten sind insbesondere die Lauffläche bzw. die Kontaktfläche in keiner Relativstellung durch Kanten unterbrochen. Mit noch anderen Worten berührt in keiner Relativstellung eine Bauteilkante die Lauffläche. So wird eine Ganghärte noch gleichmäßiger.

Gemäß einem Aspekt der Erfindung kann das medizinische Instrument als chirurgische Klemme oder eine chirurgische Zange ausgebildet sein. Bei einer chirurgischen Klemme oder Zange ist eine gleichbleibende Ganghärte von besonderem Interesse.

Gemäß einer weiteren Ausführungsform kann die erste Lauffläche und/oder die zweite Lauffläche umfangsseitig / außenseitig / konturseitig umlaufend eine Kantenverrundung / Fase aufweisen. Dies erhöht eine Bauteilstabilität, vereinfacht einen Herstellungsprozess und stellt eine gleichbleibende Reibungskraft am Umfang und damit einen gleichbleibenden Gang sicher.

Vorzugsweise kann eine Höhe des Absatzabschnitts oder des Absatzelements ausgehend vom Fuße des Absatzabschnitts oder des Absatzelements in Richtung der Schwenkachse minimal 0,1 mm und/oder maximal 1 mm, besonders bevorzugt minimal 0,3 mm und/oder maximal 0,6 mm betragen. Alternativ oder zusätzlich kann eine Höhe eines Spalts bzw. ein Spaltmaß zwischen dem ersten Lagerabschnitt und dem zweiten Lagerabschnitt in Richtung der Schwenkachse 11 minimal 0,1 mm und/oder maximal 1mm, besonders bevorzugt minimal 0,3 mm und/oder maximal 0,6 mm betragen.

Insbesondere ist der Absatzabschnitt des ersten Lagerabschnitts gleich zu dem Absatzabschnitt des zweiten Lagerabschnitts ausgebildet. So wird ein symmetrischer Aufbau bereitgestellt und auch eine Fertigung kann auf nur eine Ausführungsform des Absatzabschnitts eingestellt werden.

Vorzugsweise kann eine Rauheit/ Rauigkeit Ra der ersten Lauffläche und/oder der zweiten Lauffläche kleiner als 1µm sein, vorzugsweise kleiner als Ra 0,4 µm sein.

Vorzugsweise kann die erste und/oder zweite Lauffläche eine Fläche von mindestens 0,5cm² und/oder maximal 5cm² aufweisen.

Vorzugsweise kann ein Durchmesser einer zu der Schwenkachse koaxialen Durchgangsbohrung maximal 25% des Außendurchmessers des Absatzabschnitts oder Absatzelements betragen. Damit wird eine ausreichende Auflage- und Kontaktfläche der beiden Laufflächen sichergestellt.

Insbesondere kann die erste Lauffläche und/oder die zweite Lauffläche eine Oberflächenbearbeitung oder -beschichtung aufweisen, um eine besonders gute Laufeigenschaft zu gewährleisten. Beispielsweise kann der Absatzabschnitt und damit auch die Lauffläche ein spezielles Gefüge, wie etwa ein Martensit aufweisen, um eine hohe Festigkeit bei guter Umformung zu erhalten. Auch kann die Lauffläche eine reibungsarme Beschichtung aufweisen.

### Kurzbeschreibung der Figuren

Die Erfindung wird nachfolgend anhand einer bevorzugten Ausführungsform mit Hilfe von Figuren näher erläutert. Es zeigen:
- Fig. 1: eine perspektivische Teilansicht eines männlichen Lagerabschnitts eines erfindungsgemäßen medizinischen Instruments einer bevorzugten Ausführungsform,
- Fig. 2: eine Teilansicht einer Draufsicht auf den Lagerabschnitt des medizinischen Instruments gemäß der bevorzugten Ausführungsform,
- Fig. 3: eine perspektivische Teilansicht des medizinischen Instruments aus Fign. 1 und 2 in einem zusammengebauten Gebrauchszustand mit geschlossenen Instrumentenbranchen,
- Fig. 4: eine perspektivische Teilansicht des medizinischen Instruments aus Fig. 3, bei dem die Instrumentenbranchen auseinandergeschwenkt sind, und
- Fign. 5, 6: einen Durchsteckschluss nach Stand der Technik.

Die Figuren sind schematischer Natur und sollen nur dem Verständnis der Erfindung dienen. Gleiche Elemente sind mit denselben Bezugszeichen versehen.

### Detaillierte Beschreibung bevorzugter Ausführungsformen

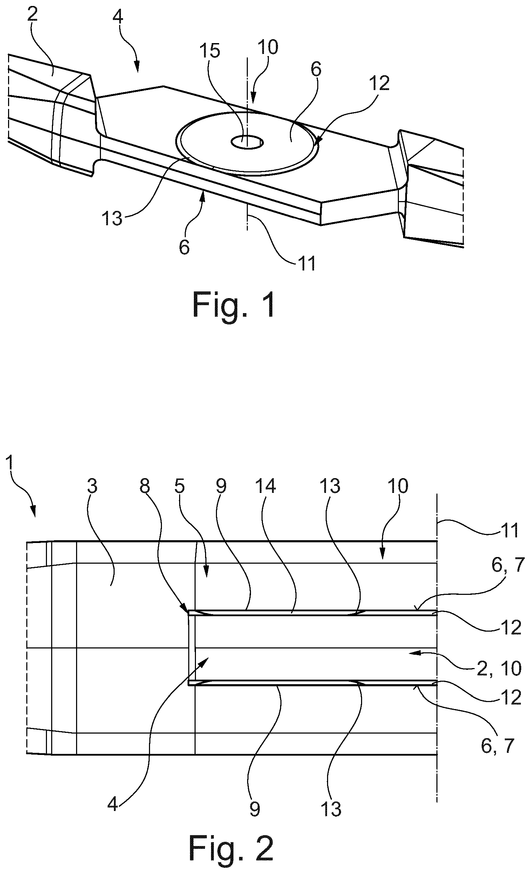

Figuren 1 bis 4 zeigen ein medizinisches Instrument 1 einer bevorzugten Ausführungsform in Form einer chirurgischen Klemme. Figur 1 zeigt dessen männliche Instrumentenbranche 2 und Fign. 2 bis 4 sowohl die männliche Instrumentenbranche 2 als auch eine zu dieser schwenkbare, weibliche Instrumentenbranche 3 im zusammengebauten, gebrauchsfertigen Zustand des Instruments 1, in welchem das Instrument 1 für einen chirurgischen Eingriff verwendbar ist und die männliche und weibliche Instrumentenbranche 2, 3 nicht mehr voneinander getrennt werden können.

Die männliche Instrumentenbranche 2 weist einen rückseitigen / endständigen, proximalen Greifabschnitt / Griffabschnitt (hier nicht dargestellt), einen frontseitigen, distalen Klemmabschnitt (hier nicht dargestellt) sowie einen zwischen diesen angeordneten männlichen Lagerabschnitt in Form eines Durchsteckteils 4 in Form eines Parallelepipeds auf. In gleicher Weise hat die weibliche Instrumentenbranche 3 einen Greifabschnitt (nicht dargestellt), einen Klemmabschnitt (nicht dargestellt) und einen zwischen diesen angeordneten weiblichen Lagerabschnitt in Form eines weiblichen Durchsteckkastens 5. Für die Lagerung weist der weibliche Durchsteckkasten 5 eine Durchsteck-/Durchgangsöffnung 8 auf, in der zwei einander zugewandte bzw. gegenüberliegende, parallele plane Seitenflächen 9 ausgebildet sind, in welche das männliche Durchsteckteil 4 durchgreift.

Wie in Fig. 2 bis 4 gezeigt, liegen die beiden Instrumentenbranchen 2, 3 an den Lagerabschnitten 4, 5 über eine männliche Lauffläche 6, die auf Seiten des männlichen Lagerabschnitts 4 ausgebildet ist und einer weiblichen Lauffläche 7, die auf Seiten des weiblichen Lagerabschnitts ausgebildet ist, flächig schwenkgleitend aneinander, so dass die männliche Instrumentenbranche 2 gegenüber der weiblichen Instrumentenbranche 3 zwischen Relativpositionen einer maximalen Öffnungsstellung, in welcher die Klemme komplett geöffnet ist und einer Schließstellung, in welcher die beiden frontseitigen Klemmabschnitte klemmend aufeinanderliegen, schwenkbar ist.

Im Unterschied zum Stand der Technik weist der in Figur 1 in Vergrößerung gezeigte männliche Lagerabschnitt 4 in einem zentralen Lager-Teilbereich 10 beidseits abgewandt einen kreisrunden, zylinderförmigen bzw. ringförmigen, sich in Richtung einer Schwenkachse 11 erstreckenden Sockel 12 als Absatzabschnitt auf. Beide einander abgewandte Sockel 12 liegen koaxial zueinander und weisen jeweils eine gleichgroße stirnseitige, plane Fläche auf, welche jeweils die männliche Lauffläche 6 bilden und einander abgewandt sowie parallel zueinander sind. Diese beiden Sockel 12 um die Schwenkachse 11 herum bzw. konzentrisch zu dieser mit abgesetzter Lauffläche 6 bewirken, dass nur dieser Teil der (stirnseitigen) Fläche des männlichen Durchsteckteils 4 als Lauffläche 6 in Kontakt mit der jeweiligen weiblichen Lauffläche 7 kommt (siehe Fig. 2). Die plane Lauffläche 6 ist dabei parallel und beabstandet zur restlichen planen Oberfläche des Lagerabschnitts 4. Durch den gegenüber dem restlichen Bereich des Lagerabschnitts 4 definiert abgesetzten Bereich der Lauffläche 6, der in Kontakt mit der weiblichen Lauffläche 7 steht, wird eine gleichmäßige Ganghärte erzielt.

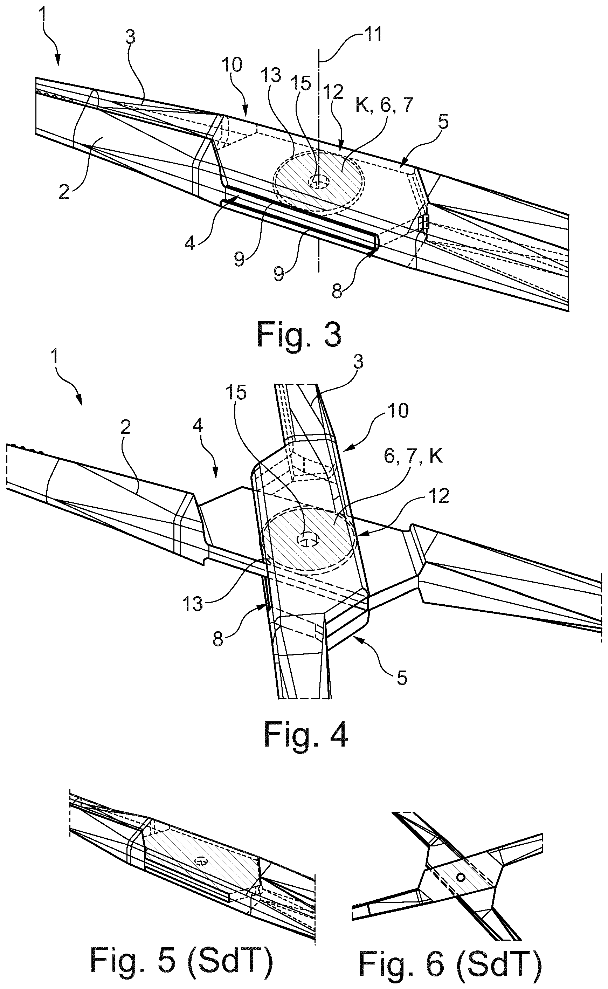

Da, wie in Fig. 3 und 4 gezeigt, die runde, männliche Lauffläche 6 stets in jeder Relativposition bzw. Relativstellung der beiden Instrumentenbranchen 2, 3 vollflächig auf der weiblichen Lauffläche 7 aufliegt, ist eine (Größer einer) Kontaktfläche K zwischen der männlichen Lauffläche 6 und der weiblichen Lauffläche 7 stets gleich groß. In Folge ist auch ein Gang im gesamten Bewegungsbereich bzw. in jeder Relativstellung der beiden Instrumentenbranchen 2, 3 zueinander gleich ausgeprägt und eine Ganghärte wird gleichmäßig gehalten. Zudem sind die Laufflächen 6, 7 bzw. ist die Kontaktfläche K in jeder Relativstellung niemals durch Kanten unterbrochen, was ebenfalls einen gleichmäßigen Gang sicherstellt. Dies resultiert aus der gleichbleibenden Kontaktfläche K und insbesondere der runden Form der abgesetzten männlichen Lauffläche 6 mit kreisrunder Außenkontur. Die männliche Lauffläche 6 liegt in jeder Relativstellung komplett auf der weiblichen Lauffläche 7 auf und kommt nie in Sicht.

An dieser Stelle sei darauf hingewiesen, dass bei den parallelen Seitenflächen /Seitenwänden 9 des Durchsteckkastens 5 nur ein mittiger bzw. zentraler Flächenteil der Seitenflächen 9 mit den kreisförmigen männlichen Laufflächen 6 in Kontakt kommt und nur diese mittigen Flächenteile für eine schwenkgleitende Bewegung auf der männlichen Lauffläche 6 aufliegen bzw. aneinander liegen. In Folge bilden natürlich auch nur diese Flächenteile die weiblichen Laufflächen 7 aus.

Um eine Schwenkbewegung, einen Gang als auch eine Reinigbarkeit weiter zu verbessern, weisen die männlichen Laufflächen 6 am Sockel 12 an ihrem kreisrunden Umfang bzw. ihrer radialen äußeren Umfangskante jeweils umlaufend eine Kantenverrundung / Fase 13 auf. Auch erstreckt sich der runde Sockel 12 (in Durchmesserrichtung, also senkrecht zur Richtung der Schwenkachse) bis an die (äußere) Kante bzw. entlang einer gesamten Breite des Durchsteckteils, um bündig mit diesem abzuschließen und eine maximal mögliche kreisförmige Auflagefläche bzw. männliche Lauffläche 6 zu bilden.

Die beiden Sockel 12 sind stoffeinstückig mit der männlichen Instrumentenbranche 2 ausgebildet und weisen nur eine sehr geringe Höhe in Richtung der Schwenkachse 11 auf, um ein Spaltmaß bzw. einen Spalt 14 zwischen dem restlichen Teil des Durchsteckteils 4 zu dem Durchsteckkasten 5 nur geringfügig zu erhöhen. Eine Höhe des Sockels 12 und damit des Spalts 14 kann insbesondere minimal 1% und/oder maximal 10% einer Dicke des Durchsteckteils 4 in Richtung der Schwenkachse 11 betragen. Insbesondere können die Sockel 12 als Absatzabschnitte additiv auf dem Durchsteckteil 4 der männlichen Instrumentenbranche 2 gefertigt sein.

Für eine positionsfeste, schwenkende Lagerung um die Schwenkachse 11 herum, weist die männliche Instrumentenbranche 2 mittig der beiden Sockel 12 bzw. koaxial zu diesen ein Durchgangsloch / Durchsteckachse / Durchgangsbohrung 15 in Richtung der Schwenkachse 11 auf. In Kombination mit komplementären, vorzugsweise zylinderförmigen, einander zugewandten Vorsprüngen (nicht dargestellt) an den parallelen Seitenflächen 9 mittig der weiblichen Lauffläche 7 wird so der männliche Lagerabschnitt durch Eingriff in das Durchgangsloch 15 schwenkbar gelagert.

Insbesondere weisen die männlichen Laufflächen 6 eine vorbestimmte Rauigkeit auf, um eine Ganghärte (leicht) zu erhöhen oder zu erniedrigen. Über die Rauigkeit kann damit die Ganghärte fein abgestimmt werden. Alternativ oder zusätzlich kann auch die weibliche Lauffläche 7 eine vorbestimmte Rauigkeit aufweisen.

Fign. 5 und 6 zeigen zum Vergleich einen Durchsteckschluss nach Stand der Technik. Bei diesen ist die Lauffläche nicht abgesetzt und es ändert sich bei einer Schwenkbewegung eine Flächengröße einer Kontaktfläche einer männlichen und weiblichen Lauffläche. In Folge ändert sich auch eine Ganghärte. Ebenso schleifen Kanten sowohl eines männlichen als auch eines weiblichen Lagerabschnitts über entsprechende Laufflächen was darüber hinaus eine Ganghärte verändert.

Im Gegensatz zum Stand der Technik bleibt, wie vorstehend erläutert, bei dem erfindungsgemäßen Instrument 1 gemäß Fig. 1 bis 4 durch den Sockel 12 mit der kreisförmigen, abgesetzten Lauffläche 6 eine Flächengröße der Kontaktfläche K für eine gleichmäßige Ganghärte stets gleich und es liegen auf den Laufflächen 6, 7 keine Kanten auf.

### Bezugszeichen

- 1: Medizinisches Instrument
- 2: Männliche Instrumentenbranche (erste Instrumentenbranche)
- 3: Weibliche Instrumentenbranche (zweite Instrumentenbranche)
- 4: Männliches Durchsteckteil (erster Lagerabschnitt)
- 5: Weiblicher Durchsteckkasten (zweiter Lagerabschnitt)
- 6: Männliche Lauffläche (erste Lauffläche)
- 7: Weibliche Lauffläche (zweite Lauffläche)
- 8: Durchgangsöffnung
- 9: parallele Seitenflächen
- 10: zentraler Lager-Teilbereich
- 11: Schwenkachse
- 12: Sockel (Absatzabschnitt)
- 13: Kantenverrundung / Fase
- 14: Spalt
- 15: Durchgangsloch

- K: Kontaktfläche

## Patentansprüche

1. Medizinisches Instrument (1) mit einer ersten Instrumentenbranche (2), die einen ersten Lagerabschnitt (4) mit zumindest einer ersten Lauffläche (6) aufweist, und mit einer zweiten Instrumentenbranche (3), die einen zweiten Lagerabschnitt (5) mit zumindest einer zweiten Lauffläche (7) aufweist, an dem die erste Lauffläche (6) flächig schwenkgleitend aufliegt, so dass die erste Instrumentenbranche (2) zu der zweiten Instrumentenbrachen (3) um eine Schwenkachse (11) schwenkbar ist, wobei
der erste Lagerabschnitt (4) und/oder der zweite Lagerabschnitt (5) im Bereich der Schwenkachse (11) einen Absatzabschnitt oder ein Absatzelement mit einer die zugehörige Lauffläche (6, 7) aufweisenden Stirnseite hat, der/das in Richtung einer Schwenkachse (11) des medizinischen Instruments (1) gegenüber dem zugehörigen Lagerabschnitt (4, 5) nach außen abgesetzt ist, so dass die zugehörige Lauffläche (6, 7) gegenüber dem zugehörigen Lagerabschnitt (4, 5) nach außen abgesetzt ist, und
die erste Lauffläche (6) und/oder die zweite Lauffläche (7) drehsymmetrisch, insbesondere kreisförmig oder ringförmig mit kreisförmigen Außendurchmesser, ausgebildet ist,
**dadurch gekennzeichnet, dass** die erste Lauffläche (6) und/oder die zweite Lauffläche (7) eine Kontaktfläche (K) bildet und in jeder Relativstellung in ihrer gesamten Fläche auf der jeweils anderen Lauffläche (6, 7) kantenlos aufliegt, derart, dass in keiner Relativstellung zueinander eine Bauteilkante der einen Lauffläche (6, 7) die andere Lauffläche (7, 6) berührt.

2. Medizinisches Instrument (1) nach Anspruch 1, **dadurch gekennzeichnet, dass** die erste Lauffläche (6) und die darauf aufliegende zweite Lauffläche (7) derart ausgebildet und aufeinander abgestimmt sind, dass bei der gleitenden Schwenkbewegung der ersten Instrumentenbranche (2) zu der zweiten Instrumentenbranche (3) eine Flächengröße der Kontaktfläche (K) der beiden aufeinanderliegenden Laufflächen (6, 7) in jeder Relativstellung gleich groß ist, um eine Ganghärte im gesamten Bewegungsbereich der Relativbewegung gleichmäßig zu halten.

3. Medizinisches Instrument (1) nach einem der vorstehenden Ansprüche, **dadurch gekennzeichnet, dass** der erste Lagerabschnitt (4) und/oder der zweite Lagerabschnitt (5) einen, insbesondere zylinderförmigen oder hohlzylinderförmigen, Sockel (12) mit einer planen, die Lauffläche (6) bildende Stirnseite als Absatzabschnitt oder Absatzelement aufweist.

4. Medizinisches Instrument (1) nach einem der vorstehenden Ansprüche, **dadurch gekennzeichnet, dass** der zumindest eine Absatzabschnitt stoffeinstückig an der zugehörigen Instrumentenbranche (2) ausgebildet ist.

5. Medizinisches Instrument (1) nach einem der vorstehenden Ansprüche, **dadurch gekennzeichnet, dass** das medizinische Instrument (1) eine Durchsteckkonfiguration aufweist, bei der der erste Lagerabschnitt (4) ein männlicher Lagerabschnitt in Form eines Durchsteckteils und der zweite Lagerabschnitt (5) ein eine Durchgangsöffnung (8) aufweisender weiblicher Lagerabschnitt in Form eines Durchsteckkastens ist, bei welcher der männliche Lagerabschnitt die Durchgangsöffnung (8) des weiblichen Lagerabschnitts durchgreift und an die zwei abgewandten Laufflächen (6) des männlichen Lagerabschnitts an zwei zugewandten Laufflächen (7) des weiblichen Lagerabschnitts schwenkgleitend anliegen.

6. Medizinisches Instrument (1) nach Anspruch 5, **dadurch gekennzeichnet, dass** der männliche Lagerabschnitt (4) zwei abgewandte, zylinderförmige oder hohlzylinderförmige, koaxial zueinander angeordnete Sockel (12) mit planer Stirnseite aufweist, welche die beiden einander abgewandten Laufflächen (6) des männlichen Lagerabschnitts (4) bilden und jeweils an der entsprechend zugewandten weiblichen Lauffläche (7) anliegen.

7. Medizinisches Instrument (1) nach einem der vorstehenden Ansprüche, **dadurch gekennzeichnet, dass** das medizinische Instrument (1) eine chirurgische Klemme oder eine chirurgische Zange ist.

8. Medizinisches Instrument (1) nach einem der vorstehenden Ansprüche, **dadurch gekennzeichnet, dass** die erste Lauffläche (6) und/oder die zweite Lauffläche (7) umfangsseitig eine Kantenverrundung aufweist.

9. Medizinisches Instrument (1) nach einem der vorstehenden Ansprüche, **dadurch gekennzeichnet, dass** eine Höhe des Absatzabschnitts oder des Absatzelements ausgehend vom Fuße des Absatzabschnitts oder des Absatzelements in Richtung der Schwenkachse (11) minimal 0,1 mm und/oder maximal 1 mm, besonders bevorzugt minimal 0,3 mm und/oder maximal 0,6 mm, beträgt und/oder dass eine Höhe eines Spalts zwischen dem ersten Lagerabschnitt (4) und dem zweiten Lagerabschnitt (5) in Richtung der Schwenkachse (11) minimal 0,1 mm und/oder maximal 1 mm, besonders bevorzugt minimal 0,3 mm und/oder maximal 0,6 mm, beträgt.

10. Medizinisches Instrument (1) nach einem der vorstehenden Ansprüche, **dadurch gekennzeichnet, dass** die erste Lauffläche (6) und/oder zweite Lauffläche (7) eine Fläche von mindestens 0,5cm² und/oder maximal 5cm² aufweisen.

11. Medizinisches Instrument (1) nach einem der vorstehenden Ansprüche, **dadurch gekennzeichnet, dass** eine Rauheit Ra der ersten Lauffläche und/oder der zweiten Lauffläche kleiner als 1µm, vorzugsweise kleiner als Ra 0,4 µm, ist.

## Claims

1. A medical instrument (1) with a first instrument branch (2), which has a first bearing portion (4) with at least one first bearing surface (6), and with a second instrument branch (3), which has a second bearing portion (5) with at least one second bearing surface (7), on which the first bearing surface (6) rests in a flat, pivot-sliding manner, so that the first instrument branch (2) is pivotable about a pivot axis (11) relative to the second instrument branch (3), wherein
the first bearing portion (4) and/or the second bearing portion (5) has, in the region of the pivot axis (11), a shoulder portion or a shoulder element with a front face comprising the associated bearing surface (6, 7), said shoulder portion or shoulder element being offset outward in the direction of a pivot axis (11) of the medical instrument (1) relative to the associated bearing portion (4, 5), so that the associated bearing surface (6, 7) is offset outward relative to the associated bearing portion (4, 5), and
the first bearing surface (6) and/or the second bearing surface (7) is formed rotationally symmetrical, in particular circular or annular with a circular outer diameter,
**characterized in that** the first bearing surface (6) and/or the second bearing surface (7) forms a contact surface (K) and rests in its entire surface on the respective other bearing surface (6, 7) without edges in each relative orientation, such that, in any relative position to each other, a component edge of one bearing surface (6, 7) does not touch the other bearing surface (7, 6).

2. The medical instrument (1) according to claim 1, **characterized in that** the first bearing surface (6) and the second bearing surface (7) resting thereon are configured and correlated with each other such that during the sliding pivoting movement of the first instrument branch (2) to the second instrument branch (3), an area size of the contact surface (K) of the two bearing surfaces (6, 7) lying on top of each other is the same size in each relative orientation in order to maintain a smoothness of action uniformly in the entire range of movement of the relative movement.

3. The medical instrument (1) according to one of the preceding claims, **characterized in that** the first bearing portion (4) and/or the second bearing portion (5) comprises a pedestal (12), in particular cylindrical or hollow-cylindrical, having a planar front face forming the bearing surface (6) as shoulder portion or shoulder element.

4. The medical instrument (1) according to one of the preceding claims, **characterized in that** the at least one shoulder portion is integrally formed on the associated instrument branch (2).

5. The medical instrument (1) according to one of the preceding claims, **characterized in that** the medical instrument (1) has a push-through configuration in which the first bearing portion (4) is a male bearing portion in the form of a push-through part and the second bearing portion (5) is a female bearing portion in the form of a push-through box having a passage opening (8), in which the male bearing portion engages through the passage opening (8) of the female bearing portion and pivotably slides against the two averted bearing surfaces (6) of the male bearing portion against two facing bearing surfaces (7) of the female bearing portion.

6. The medical instrument (1) according to claim 5, **characterized in that** the male bearing portion (4) has two cylindrical or hollow cylindrical pedestals (12) facing away from each other, coaxially arranged with respect to each other and with a planar front face, which form the two bearing surfaces (6) of the male bearing portion (4) facing away from each other and each abut the corresponding facing female bearing surface (7).

7. The medical instrument (1) according to one of the preceding claims, **characterized in that** the medical instrument (1) is a surgical clamp or surgical forceps.

8. The medical instrument (1) according to one of the preceding claims, **characterized in that** the first bearing surface (6) and/or the second bearing surface (7) has a rounded edge around the circumference.

9. The medical instrument (1) according to one of the preceding claims, **characterized in that** a height of the shoulder portion or of the shoulder element starting from the foot of the shoulder portion or of the shoulder element in the direction of the pivot axis (11) is a minimum of 0.1 mm and/or a maximum of 1 mm, particularly preferably a minimum of 0.3 mm and/or a maximum of 0.6 mm, and/or **in that** a height of a gap between the first bearing portion (4) and the second bearing portion (5) in the direction of the pivot axis (11) is a minimum of 0.1 mm and/or a maximum of 1 mm, particularly preferably a minimum of 0.3 mm and/or a maximum of 0.6 mm.

10. The medical instrument (1) according to one of the preceding claims, **characterized in that** the first bearing surface (6) and/or second bearing surface (7) have an area of at least 0.5cm² and/or at most 5cm².

11. The medical instrument (1) according to one of the preceding claims, **characterized in that** a roughness Ra of the first bearing surface and/or of the second bearing surface is smaller than 1µm, preferably smaller than Ra 0.4 µm.

## Revendications

1. Instrument médical (1) avec une première branche d'instrument (2) qui présente une première section de palier (4) avec au moins une première surface de roulement (6) et avec une seconde branche d'instrument (3) qui présente une seconde section de palier (5) avec au moins une seconde surface de roulement (7) au niveau de laquelle section la première surface de roulement (6) repose à plat de manière glissante et pivotante, de sorte que la première branche d'instrument (2) soit pivotante par rapport à la seconde branche d'instrument (3) autour d'un axe de pivotement (11), dans lequel
la première section de palier (4) et/ou la seconde section de palier (5) présente dans la zone de l'axe de pivotement (11) une section d'épaulement ou un élément d'épaulement avec un côté avant présentant la surface de roulement (6, 7) afférente, section ou épaulement qui est étagé vers l'extérieur en direction d'un axe de pivotement (11) de l'instrument (1) médical par rapport à la section de palier (4, 5) afférente, de sorte que la surface de roulement (6, 7) afférente soit étagée vers l'extérieur par rapport à la section de palier (4, 5) afférente et
la première surface de roulement (6) et/ou la seconde surface de roulement (7) est formée de manière symétrique en rotation, en particulier en cercle ou en anneau avec un diamètre extérieur circulaire,
**caractérisé en ce que** la première surface de roulement (6) et/ou la seconde surface de roulement (7) forme une surface de contact (K) et repose sans arête dans chaque position relative dans leur surface entière sur l'autre surface de roulement (6, 7) respective de telle manière qu'une arête de composant de l'une surface de roulement (6, 7) ne touche l'autre surface de roulement (7, 6) dans aucune position relative l'une par rapport à l'autre.

2. Instrument médical (1) selon la revendication 1, **caractérisé en ce que** la première surface de roulement (6) et la seconde surface de roulement (7) reposant dessus sont formées et adaptées l'une à l'autre de sorte que lors du mouvement pivotant glissant de la première branche d'instrument (2) par rapport à la seconde branche d'instrument (3), une taille de surface de la surface de contact (K) des deux surfaces de roulement (6, 7) reposant l'une sur l'autre est de même taille dans chaque position relative afin de maintenir une dureté de pas uniforme sur la zone de mouvement entière du mouvement relatif.

3. Instrument médical (1) selon l'une quelconque des revendications précédentes, **caractérisé en ce que** la première section de palier (4) et/ou la seconde section de palier (5) présente un socle (12), en particulier cylindrique ou cylindrique et creux avec un côté avant plat formant la surface de roulement (6) comme section d'épaulement ou élément d'épaulement.

4. Instrument médical (1) selon l'une quelconque des revendications précédentes, **caractérisé en ce que** la au moins une section d'épaulement est formée en un seul matériau au niveau de la branche d'instrument (2) afférente.

5. Instrument médical (1) selon l'une quelconque des revendications précédentes, **caractérisé en ce que** l'instrument médical (1) présente une configuration d'enfichage pour laquelle la première section de palier (4) est une section de palier mâle en forme de partie enfichée et la seconde section de palier (5) est une section de palier femelle présentant une ouverture traversante (8) en forme de caisse enfichée pour laquelle la section de palier mâle traverse l'ouverture traversante (8) de la section de palier femelle et contre laquelle reposent de manière glissante et pivotantes deux surfaces de roulement (6) éloignées de la section de palier mâle au niveau de deux surfaces de roulement (7) tournées vers la section de palier femelle.

6. Instrument médical (1) selon la revendication 5, **caractérisé en ce que** la section de palier (4) mâle présente deux socles (12) éloignés, cylindriques ou cylindriques et creux, agencés coaxialement l'un à l'autre avec un côté avant plat qui forment les deux surfaces de roulement (6) éloignés l'une de l'autre de la section de palier (4) mâle et reposent respectivement contre la surface de roulement (7) femelle tournée de manière correspondante.

7. Instrument médical (1) selon l'une quelconque des revendications précédentes, **caractérisé en ce que** l'instrument médical (1) est un clamp chirurgical ou une pince chirurgicale.

8. Instrument médical (1) selon l'une quelconque des revendications précédentes, **caractérisé en ce que** la première surface de roulement (6) et/ou la seconde surface de roulement (7) présente un arrondi d'arête côté circonférence.

9. Instrument médical (1) selon l'une quelconque des revendications précédentes, **caractérisé en ce qu'** une hauteur de la section d'épaulement ou de l'élément d'épaulement s'élève au minimum à 0,1 mm et/ou au maximum à 1 mm, le plus préférentiellement au minimum à 0,3 mm et/ou au maximum à 0,6 mm en partant du pied de la section d'épaulement ou de l'élément d'épaulement en direction de l'axe de pivotement (11), et/ou **en ce qu'**une hauteur d'une fente entre la première section de palier (4) et la seconde section de palier (5) s'élève au minimum à 0,1 mm et/ou au maximum à 1 mm, le plus préférentiellement au minimum à 0,3 mm et/ou au maximum à 0,6 mm en direction de l'axe de pivotement (11).

10. Instrument médical (1) selon l'une quelconque des revendications précédentes, **caractérisé en ce que** la première surface de roulement (6) et/ou la seconde surface de roulement (7) présente une surface d'au moins 0,5 cm² et/ou au maximum de 5 cm².

11. Instrument médical (1) selon l'une quelconque des revendications précédentes, **caractérisé en ce qu'** une rugosité Ra de la première surface de roulement et/ou de la seconde surface de roulement est inférieure à 1 µm, de préférence inférieure à une Ra de 0,4 µm.
